# EUROPÄISCHE PATENTSCHRIFT

(11) **EP 1 847 259 B1**
(45) Veröffentlichungstag und Bekanntmachung des Hinweises auf die Patenterteilung: **25.11.2009**
(21) Anmeldenummer: 06007842.5
(22) Anmeldetag: 13.04.2006
(51) Int. Cl.: A61K 9/20

(54) **Poloxamere als Schmiermittel für pharmazeutische Zusammensetzungen enthaltend Thieno[3,2-c]pyridin-Derivate**
Compositions comprising thieno[2,3-c]pyridine derivatives as active agents with poloxamers as lubricants
Compositions pharmaceutiques comprenant derivés du thieno[2,3-c]pyridine et des poloxamères comme lubrifiants

(43) Veröffentlichungstag der Anmeldung: 24.10.2007
(73) Patentinhaber: Rentschler Pharma GmbH, 88471 Laupheim (DE)
(72) Erfinder: Beckert, Thomas Dr., 88447 Warthausen-Birkenhard (DE); Braun, Michael Dr., 89250 Senden (DE); Neuer, Klaus, 88477 Schwendi-Orsenhausen (DE)
(74) Vertreter: Westendorp | Sommer

(56) Entgegenhaltungen:
- EP-A- 1 005 864
- WO-A-00/01364
- WO-A-00/10534
- WO-A-2005/048992

## Beschreibung

Die Erfindung betrifft stabile Formulierungen enthaltend Thieno[3,2-c]pyridin-Derivate als Wirkstoffe, im besonderen Clopidogrelhydrogensulfat oder andere pharmazeutisch annehmbare Salze.

Thieno[3,2-c]pyridin-Derivate sind eine bekannte Wirkstoffklasse, die insbesondere als Thrombozyten-Aggregationshemmer eingesetzt werden, zu der unter anderem Clopidogrel und Ticlopidin zählen. Diese Wirkstoffe werden unter anderem in Tabletten formuliert, wobei geeignete Schmier- und Formentrennmittel nötig sind. Schmier- und Formentrennmittel sind Hilfsstoffe, die die interpartikuläre Reibung während der Kompression reduzieren und die Reibung zwischen Tablette und Matrizenwand während des Ausstoßens herabsetzen sowie ein Kleben an den Stempelwerkzeugen der Tablettenmaschine verhindern sollen, teilweise auch in Füllmassen für HartgelatineKapseln, wenn bei der Abfüllung eine Komprimierung erfolgt.

Schnell freisetzende pharmazeutische Tablettenformulierungen sollen neben einer hohen Zerfallsgeschwindigkeit im Allgemeinen auch eine ausreichend hohe Bruchfestigkeit aufweisen. Eine entsprechende mechanische Stabilität gewährleistet eine unkomplizierte Weiterverarbeitung zum Beispiel bei der Konfektionierung und trägt zur Arzneimittelsicherheit bei. Beide Eigenschaften werden, zum Teil in entscheidender Weise, durch das verwendete Schmier- und Formentrennmittel beeinflusst.

Thieno[3,2-c]pyridin-Derivate sind bekannt dafür, gegenüber zahlreichen gängigen pharmazeutischen Hilfsstoffen wie Gelatine oder Povidon empfindlich zu reagieren, d.h. abgebaut zu werden.

Vor allem das basisch reagierende Standardschmiermittel Magnesiumstearat bewirkt signifikanten Wirkstoffabbau in der Tablettenformulierung. Dieses Problem ist bekannt und wird zum Beispiel in US 4,591,592 beschrieben. Das Problem kann durch Zusätze von organischen Säuren minimiert werden (siehe zum Beispiel US 4,591,592), dies gestaltet jedoch die Formulierungen unerwünscht komplex. Weiterhin wurden verschiedene Alternativen für das Standardschmiermittel Magnesiumstearat vorgeschlagen, um einen Wirkstoffabbau zu verhindern, und so die Stabilität der Formulierung zu gewährleisten.

US 5,520,928 betrifft die Stabilisierung einer pharmazeutischen Zusammensetzung, die das Thieno[3,2-c]pyridin-Derivat Ticlopidinhydrochlorid enthält. Die Stabilisierung wird durch die Verwendung von Stearinsäure erreicht, die auch als Schmiermittel dient. Des Weiteren umfasst die pharmazeutische Zusammensetzung gemäß US 5,520,928 mindestens einen Hilfsstoff ausgewählt aus Desintegrationsmittel, Bindemittel und Verdünner, die im Wesentlichen frei von organischen Säuren sind, mit Ausnahme von Stearinsäure.

WO 2005/070464 betrifft eine stabile pharmazeutische Zusammensetzung enthaltend als Wirkstoff Clopidogrelhydrogensulfat. Als Schmiermittel werden verschiedene Alternativen zum Standardschmiermittel Magnesiumstearat offenbart. Neben Natriumcarboxylmethylstärke wird hydriertes Pflanzenöl als Schmiermittel offenbart.

EP 1 310 245 betrifft ebenfalls pharmazeutische Zusammensetzungen in der Form von Tabletten, umfassend Clopidogrelhydrogensulfat sowie ein Schmiermittel. Um die Stabilität der Zusammensetzung zu erhöhen, werden als Alternative zu den Schmiermitteln Magnesiumstearat und Calciumstearat die Schmiermittel Zinkstearat, Stearinsäure und Natriumstearylfumarat vorgeschlagen.

WO 00/01364 betrifft eine stabile pharmazeutische Zusammensetzung umfassend Thieno[3,2-c]pyridin-Derivate sowie als wasserlösliches hydrophiles Schmiermittel Polyethylenglykol und mindestens einen pharmazeutisch annehmbaren Hilfsstoff. Ein bevorzugtes Thieno[3,2-c]pyridin-Derivat ist Ticlopidin oder Ticlopidinhydrochlorid. Die pharmazeutische Zusammensetzung ist im Wesentlichen frei von Magnesiumstearat, wasserlöslichem Polyvinylpyrrolidon und Natriumstärkeglycolat.

WO 2005048992 offenbart einen weiteren Lösungsansatz, in dem mit Polyvinylacetat oder Polyvinylalkoholat überzogene Wirkstoffpartikel, -granulate oder -agglomerate eingesetzt werden. Um eine erhöhte Stabilität des Wirkstoffs zu erhalten, wird als Schmiermittel hydriertes Pflanzenöl vorgeschlagen. Statt Hydrogensulfat werden andere pharmazeutisch geeignete Salze, wie das Mesilat, Hydrobromid oder Hydrochlorid eingesetzt.

WO 2004098593 betrifft eine stabile, nicht-hygroskopische pharmazeutische Zusammensetzung, die amorphes Clopidogrel enthält. Weiterhin umfasst die Zusammensetzung Calcium- oder Magnesiumstearat sowie einen nicht-hygroskopischen Zusatzstoff mit mindestens einem weiteren Hilfsstoff.

WO 00/10534 offenbart eine wässrige Thieno [3,2-c] pyridin Zubereitung auf Basis eines Lyophilisats.

Vor diesem Hintergrund wurde ein neues effektives Schmiermittel gesucht, mit dessen Hilfe auf einfachem Weg stabile pharmazeutische Zusammensetzungen enthaltend Thieno[3,2-c]pyridin-Derivate hergestellt werden können, wobei Vorteile wie z.B. eine erhöhte Zerfallsgeschwindigkeit bei einer schnell freisetzenden pharmazeutischen Tablettenformulierung und eine erhöhte Bruchfestigkeit erhalten werden.

Aufgabe der Erfindung ist es, ein Schmiermittel für eine pharmazeutische Zusammensetzung enthaltend ein Thieno[3,2-c]pyridin-Derivat bereitzustellen, so dass sich ein schneller Zerfall der Zusammensetzung und/oder eine erhöhte Bruchfestigkeit zeigt.

Vorstehende Aufgabe wird durch die Gegenstände der unabhängigen Ansprüche 1, und 5 - 7 gelöst. Vorteilhafte Weiterentwicklungen gehen aus den Unteransprüchen hervor.

Vorteilhaft wird gemäß der Erfindung ein neues Schmiermittel eingesetzt. Es werden Poloxamere, wie insbesondere Poloxamer 188 oder Poloxamer 407 als Schmiermittel verwendet.

Vorteilhaft enthält eine pharmazeutische Zusammensetzung:
ein Thieno[3,2-c]pyridin-Derivat als Wirkstoff und
ein Poloxamer.

Die pharmazeutische Zusammensetzung kann als Tablette, Mikrotablette, Nanotablette, als in Kapseln gefüllte Mikrotabletten, Nanotabletten, oder als in Sachets gefüllte(s) Mikrotabletten, Nanotabletten, vorliegen, jedoch vorzugsweise als Tablette.

Weiterhin betrifft die Erfindung die Verwendung eines Schmiermittels, ausgewählt aus der Gruppe der Blockcopolymere aus Ethylenoxid und Propylenoxid bzw. ein Poloxamer, zur Herstellung einer pharmazeutischen Zusammensetzung, wobei die pharmazeutische Zusammensetzung ein Thieno[3,2-c]pyridin-Derivat enthält.

Die Erfindung betrifft auch ein Verfahren zur Herstellung einer pharmazeutischen Zusammensetzung, enthaltend ein Thieno[3,2-c]pyridin-Derivat, bei der als Schmiermittel ein Blockcopolymer aus Ethylenoxid und Propylenoxid bzw. ein Poloxamer verwendet wird.

Das Thieno[3,2-c]pyridin-Derivat (im Folgenden auch Wirkstoff bezeichnet) kann als freie Base vorliegen oder als ein beliebiges pharmazeutisch annehmbares Salz davon. Sofern der Wirkstoff als Salz vorliegt, ist das Anion bevorzugter Weise ausgewählt aus der Gruppe, bestehend aus Sulfat, Hydrogensulfat, Bromid, Chlorid, Iodid, Mesilat, Carbonat und Hydrogencarbonat. Das Thieno[3,2-c]pyridin-Derivat ist vorzugsweise ausgewählt aus der Gruppe, bestehend aus Clopidogrel und Ticlopidin sowie Mischungen davon.

Poloxomer ist der internationale Freiname für Blockcopolymere aus Ethylenoxid und Propylenoxid.

Das Poloxamer ist bevorzugt ein Blockcopolymer wie es unter dem Handelsnamen Pluronic oder Lµtrol verkauft wird. Hierbei handelt es sich um Verbindungen der Formel:

HO(C₂H₄O)ₐ(C₃H₆O)_{b}(C₂H₄O)ₐH

Bevorzugter Weise ist hier bei a im Bereich von 8 bis 16 und b im Bereich von 16 bis 24, a im Bereich von 74 bis 84 und b im Bereich von 23 bis 32, a im Bereich von 59 bis 69 und b im Bereich von 32 bis 42, a im Bereich von 131 bis 151 und b im Bereich von 39 bis 49, a im Bereich von 91 bis 111 und b im Bereich von 51 bis 61.

Stärker bevorzugter Weise ist hierbei a 12 und b 20 (Poloxamer 124), a 79 bzw. 80 und b 27 bzw. 28 (Poloxamer 188), a 64 und b 37 (Poloxamer 237), a 141 und b 44 (Poloxamer 338), a 101 und b 56 (Poloxamer 407). Am stärksten bevorzugt ist das Polyethylenoxid / Propylenoxid-Copolymer Poloxamer 188 und/oder Poloxamer 407, die unter den Handelsnamen Lµtrol micro 68 oder Lµtrol micro 127 verkauft werden.

Das Poloxamer ist in der pharmazeutischen Zusammensetzung in einer Menge von 0,1 bis 25 Gew.%, bevorzugt 6 bis 17 Gew.%, stärker bevorzugt 10 bis 15 Gew.% und am stärksten bevorzugt 12 bis 13 Gew.%, bezogen auf das Gesamtgewicht der pharmazeutischen Zusammensetzung enthalten.

Bevorzugte Untergrenzen für den Gehalt des Poloxamers in der pharmazeutischen Zusammensetzung sind 0,1, 6, 10 und 12 Gew.%. Bevorzugte Obergrenzen für den Gehalt des Poloxamers in der pharmazeutischen Zusammensetzung sind 25, 17, 15, 13 Gew.%.

Durch die Verwendung der hydrophilen und oberflächenaktiven Poloxamere als Schmiermittel lassen sich mechanisch sehr stabile Konprimate herstellen, ohne die Zerfallszeit oder die Wirkstofffreigabe aus der Tablette negativ zu beeinflussen. Dies ist im vorliegenden Falle besonders wichtig, da Thieno[3,2-c]pyridin-Derivate wie Clopidogrel enthaltende Tablettenkerne bevorzugt mit Isolierfilmen wie z.B. Opadry®AMB oder EUDRAGIT® E PO überzogen werden.

Die pharmazeutische Zusammensetzung ist bevorzugter Weise eine schnell freisetzende pharmazeutische Tablettenformulierung. Diese kann neben dem Wirkstoff einen oder mehrere Füll- und Bindemittel, Zerfallhilfsmittel, Schmier- und Formentrennmittel sowie Fließregulierungsmittel enthalten. Es kann zum Beispiel mikrokristalline Zellulose als gängiger Füllstoff mit sehr guter plastischer Verformbarkeit zusammen mit dem Trockenbindemittel Copovidon eingesetzt werden, wobei gute Bruchfestigkeits- und Abriebswerte erzielt werden. Mannitol als löslicher Zuckeralkohol gewährleistet zusammen mit dem effektiven Zerfallsförderer Crospovidon ein schnelles Quellen und Auflösen der Tablette, um den Wirkstoff rasch zu liberieren. Es können aber auch andere Füll- und Bindemittel oder Zerfallhilfsmittel mit vergleichbaren Eigenschaften eingesetzt werden.

Das Schmiermittel der Erfindung ermöglicht es, auf einfachem Weg stabile Tablettenzubereitungen mit Thieno[3,2c]pyridin-Derivaten herzustellen, wobei die bei hydrophoben Schmiermitteln oft beobachteten Nachteile, wie verlangsamter Tablettenzerfall und/oder verlangsamte Wirksoffliberation nicht beobachtet werden. Überraschenderweise konnte festgestellt werden, dass Tabletten mit einem Poloxamer als Schmiermittel viel stabiler sind als Tabletten mit äquivalentem Zusatz an Magnesiumstearat. Der Wirkstoffabbau verläuft etwa gleich langsam wie bei Tabletten mit hydriertem Pflanzenöl als Schmier- und Formentrennmittel, jedoch werden die genannten Nachteile vermieden. Gegenüber Magnesiumstearat und hydriertem Pflanzenöl zeigten die Tablettenkerne eine für die spätere Befilmung erwünschte höhere Bruch- und Abriebfestigkeit, ohne dass es dabei zu einem schnellen Abbau des Wirkstoffs gekommen wäre. Diese vorteilhaften Eigenschaften der Schmiermittel der Erfindung werden in den Beispielen demonstriert.

Im Folgenden erfolgt eine detaillierte Beschreibung der Erfindung anhand von Beispielen. Dabei werden weitere Merkmale der Erfindung offenbart, die untereinander kombiniert werden können.

### Beispiele

### Beispiel 1

111,8 g Clopidogrelhydrogensulfat,
41,2 g Mannitol DC,
79,9 g MCC 102,
37,1 g MCC 200,
4,3 g Copovidon,
10,3 g Crospovidon,
14,3 g Poloxamer 188,
1,1 g hochdisperses Silicuiumdioxid
werden in einem Laborfreifallmischer gemischt und anschließend zu runden Tabletten mit einem Durchmesser von 9 mm verpresst.

### Beispiel 2

Wie Beispiel 1, nur dass anstatt Poloxamer 188 Magnesiumstearat verwendet wurde.

### Beispiel 3

Wie Beispiel 1, nur dass anstatt Poloxamer 188 hydriertes Pflanzenöl verwendet wurde.

Tabelle 1 gibt einen Überblick über die Zusammensetzungen der nach Beispielen 1 - 3 hergestellten Tabletten.

**Tabelle 1**

| **Komponente** | **Menge [mg / DF]** | **Menge [mg / DF]** | **Menge [mg / DF]** | **Funktion** |
|---|---|---|---|---|
| | **Bsp 1** | **Bsp 2** | **Bsp 3** | |
| Clopidogrelhydrogensulfat | 111,8 | 111,8 | 111,8 | Aktive Komponente |
| Mannitol DC | 41,2 | 41,2 | 41,2 | Füllstoff/Bindemittel |
| MCC 102 | 79,9 | 79,9 | 79,9 | Füllstoff/Bindemittel |
| MCC 200 | 37,1 | 37,1 | 37,1 | Füllstoff/Bindemittel |
| Copovidon | 4,3 | 4,3 | 4,3 | Trockenbindemittel |
| Crospovidon | 10,3 | 10,3 | 10,3 | Zerfallsförderer |
| Magnesiumstearat pfl. | | 14,3 | | Schmier- u. Trennmittel |
| Hydriertes Pflanzenöl | | | 14,3 | Schmier- u. Trennmittel |
| Poloxamer 188 | 14,3 | | | |
| Siliciumdioxid, hochdisp. | 1,1 | 1,1 | 1,1 | Fliessregulierungsmittel |
| **Summe** | **300** | **300** | **300** | |

### Physikalische Charakterisierung

Die diametrale Bruchfestigkeit und die Zerfallszeit der Tabletten wurden in Übereinstimmung mit den entsprechenden Apparaturen und Methoden des Europäischen Arzneibuchs bestimmt.

Tabelle 2 zeigt die Bruchfestigkeit und die Zerfallsgeschwindigkeit der in Beispiel 1 - 3 hergestellten Tablettenformulierungen. Die Ergebnisse zeigen, dass durch die Schmiermittel der Erfindung deutlich erhöhte Bruchfestigkeiten bei beschleunigter Zerfallsgeschwindigkeiten erhalten werden.

**Tabelle 2**

| **Eigenschaft** | **Beispiel 1** | **Beispiel 2** | **Beispiel 3** |
|---|---|---|---|
| Bruchfestigkeit [N] (n=10) | ~120 | ~105 | ∼80 |
| Zerfall [min] | 4 bis 5 | 5 bis 7 | 5 bis 7 |

### Stabilitätsergebnisse

Die in den Beispielen 1 - 3 hergestellten Tabletten wurden in PVC-Alu-Folie eingeblistert und bei 60°C in einem Klimaschrank eingelagert. Nach jeweils zwei und vier Wochen wurden entsprechende Proben mittels einer geeigneten HPLC-Methode untersucht. Zu Vergleichszwecken wurde der unter gleichen Bedingungen eingelagerte reine Wirkstoff zu den entsprechenden Prüfzeitpunkten ebenfalls mit untersucht.

Die entstandenen, aufsummierten Abbauprodukte werden in Tabelle 3 in Prozent, bezogen auf den initialen Wirkstoffgehalt angegeben.

**Tabelle 3**

| **Untersuchungszeitpunkt** | **Clopidogrelhydrogensulfat reiner Wirkstoff** | **Formulierung nach Beispiel 1** | **Formulierung nach Beispiel 2** | **Formulierung nach Beispiel 3** |
|---|---|---|---|---|
| Ausgangswert | 0,92% | 1,19% | 1,20% | 1,18% |
| 2 Wochen | 0,99% | 1,42% | 3,10% | 1,44% |
| 4 Wochen | 1,19% | 1,62% | 4,19% | 1,65% |

Aus der Tabelle geht eindeutig hervor, dass bei Verwendung von Poloxamer als Schmiermittel ein gegenüber Magnesiumstearat deutlich besseres und gegenüber hydriertem Pflanzenöl ein vergleichbares Ergebnis erzielt werden kann.

## Patentansprüche

1. Pharmazeutische Zusammensetzung enthaltend
a) ein Thieno[3,2-c]pyridin-Derivat als Wirkstoff und
b) ein Poloxamer,
wobei die pharmazeutische Zusammensetzung als Tablette, Mikrotablette, Nanotablette, als in Kapseln gefüllte Mikrotabletten und Nanotabletten oder als in Sacchets gefüllte Mikrotabletten und Nanotabletten vorliegt.

2. Pharmazeutische Zusammensetzung nach Anspruch 1, wobei das Poloxamer in einer Menge von 0,1 - 25 Gew.%, bevorzugt 6 - 17 Gew.%, stärker bevorzugt 10 - 15 Gew.% und am stärksten bevorzugt 12 - 13 Gew.%, bezogen auf das Gesamtgewicht der pharmazeutischen Zusammensetzung enthalten ist.

3. Pharmazeutische Zusammensetzung nach Anspruch 1, wobei es sich bei dem Poloxamer um Poloxamer 188 und / oder Poloxamer 407 handelt.

4. Verwendung eines Poloxamers als Schmiermittel zur Herstellung einer pharmazeutischen Zusammensetzung, enthaltend ein Thieno[3,2-c]pyridin-Derivat, wobei die pharmazeutische Zusammensetzung als Tablette, Mikrotablette, Nanotablette, als in Kapseln gefüllte Mikrotabletten und Nanotabletten oder als in Sacchets gefüllte Mikrotabletten und Nanotabletten, vorliegt.

5. Verwendung eines Poloxamers zur Herstellung einer pharmazeutischen Zusammensetzung nach Anspruch 1.

6. Verfahren zur Herstellung einer pharmazeutischen Zusammensetzung, enthaltend ein Thieno[3,2-c]pyridin-Derivat, bei dem als Schmiermittel ein Poloxamer verwendet wird, wobei die pharmazeutische Zusammensetzung als Tablette, Mikrotablette, Nanotablette, als in Kapseln gefüllte Mikrotabletten und Nanotabletten, oder als in Sacchets gefüllte Mikrotabletten und Nanotabletten, vorliegt.

## Claims

1. A pharmaceutical composition containing
a) a thieno[3,2-c]pyridine derivative as an active agent and
b) a poloxamer,
wherein the pharmaceutical composition is present as a tablet, microtablet, nanotablet, as microtablet-filled and nanotablet-filled capsules or as microtablet-filled and nanotablet-filled sachets.

2. A pharmaceutical composition according to Claim 1, wherein the poloxamer is contained in a quantity of 0.1 to 25 % by weight, preferably 6 to 17 % by weight, more preferably 10 to 15 % by weight and most preferably 12 to 13 % by weight, in relation to the total weight of the pharmaceutical composition.

3. A pharmaceutical composition according to Claim 1, wherein the poloxamer is poloxamer 188 and/or poloxamer 407.

4. Use of a poloxamer as lubricant for the preparation of a pharmaceutical composition, containing thieno[3,2-c]pyridine derivative, wherein the pharmaceutical composition is present as a tablet, microtablet, nanotablet, as microtablet-filled and nanotablet-filled capsules or as microtablet-filled and nanotablet-filled sachets.

5. Use of a poloxamer for the preparation of a pharmaceutical composition according to Claim 1.

6. A method for the preparation of a pharmaceutical composition, containing a thieno[3,2-c]pyridine derivative, in which a poloxamer is used as lubricant, wherein the pharmaceutical composition is present as a tablet, microtablet, nanotablet, as microtablet-filled and nanotablet-filled capsules or as microtablet-filled and nanotablet-filled sachets.

## Revendications

1. Composition pharmaceutique contenant
a) un dérivé thiéno[3,2-c]pyridine comme agent actif, et
b) un poloxamère,
où la composition pharmaceutique se présente sous la forme de comprimés, microcomprimés, nanocomprimés, sous la forme de capsules remplies de microcomprimés et de nanocomprimés, ou sous la forme de sachets remplis des microcomprimés et des nanocomprimés.

2. Composition pharmaceutique selon la revendication 1, où le poloxamère est présent en une quantité de 0,1 à 25% en poids, de préférence de 6 à 17% en poids, de manière plus préférée de 10 à 15% en poids et de manière la plus préférée, de 12 à 13% en poids, sur la base du poids total de la composition pharmaceutique.

3. Composition pharmaceutique selon la revendication 1, où le poloxamère est le poloxamère 188 et/ou le poloxamère 407.

4. Utilisation d'un poloxamère en tant que lubrifiant, pour la fabrication d'une composition pharmaceutique, contenant un dérivé thiéno[3,2-c]pyridine, où la composition pharmaceutique se présente sous la forme de comprimés, microcomprimés, nanocomprimés, sous la forme de capsules remplies de microcomprimés et de nanocomprimés, ou sous la forme de sachets remplis des microcomprimés et des nanocomprimés.

5. Utilisation d'un poloxamère pour la fabrication d'une composition pharmaceutique selon la revendication 1.

6. Procédé de préparation d'une composition pharmaceutique, contenant un dérivé thiéno[3,2-c]pyridine, dans lequel on utilise un poloxamère comme lubrifiant, où la composition pharmaceutique se présente sous la forme de comprimés, microcomprimés, nanocomprimés, sous la forme de capsules remplies de microcomprimés et de nanocomprimés, ou sous la forme de sachets remplis des microcomprimés et des nanocomprimés.
